# EUROPÄISCHE PATENTSCHRIFT

(11) **EP 3 290 068 B1**
(45) Veröffentlichungstag und Bekanntmachung des Hinweises auf die Patenterteilung: **30.10.2019**
(21) Anmeldenummer: 17187369.8
(22) Anmeldetag: 22.08.2017
(51) Int. Cl.: A61M 1/16, B01D 29/03, B01F 1/00

(54) **KARTUSCHENFÖRMIGER BEHÄLTER MIT EINER SIEBARTIGEN FILTEREINRICHTUNG**
CARTRIDGE-SHAPED CONTAINER WITH A SCREEN-STYLE FILTER DEVICE
CONTENANT EN FORME DE CARTOUCHE AVEC DISPOSITIF DE FILTRATION DU TYPE TAMIS

(30) Priorität: 30.08.2016 DE 102016116097
(43) Veröffentlichungstag der Anmeldung: 07.03.2018
(73) Patentinhaber: B. Braun Avitum AG, 34212 Melsungen (DE)
(72) Erfinder: KLAUS, Dinah, 48147 Münster (DE); WESSELER, Matthias, 49326 Melle (DE)
(74) Vertreter: Winter, Brandl, Fürniss, Hübner, Röss, Kaiser, Polte - Partnerschaft mbB

(56) Entgegenhaltungen:
- EP-A1- 0 019 794
- EP-A1- 2 832 339
- EP-B1- 1 610 842
- WO-A1-2005/118485
- US-A1- 2014 124 434

## Beschreibung

Die Erfindung betrifft einen kartuschenförmigen Behälter/ eine Kartusche für eine extrakorporale Blutbehandlungsmaschine, welcher/ welche mindestens einen einen Fluideinlass und/oder einen Fluidauslass bildenden Anschlussport/ Konnektor und mindestens eine an dem zumindest einen Anschlussport angeordnete, siebartige Filtereinrichtung aufweist, welche an dem Anschlussport/ Konnektor des Behälters/ der Kartusche angeordnet ist und zum Zurückhalten von sich in dem Behälter/ der Kartusche befindlichen ungelösten Pulver-/ und/ oder Feststoffpartikeln ausgelegt ist und welche mindestens zwei Öffnungen aufweist.

Der Therapieerfolg der verschiedensten Dialyseverfahren beruht unter anderem auf dem Einsatz verschiedener Puffersubstanzen, mit Hilfe derer ein veränderter Säure-Basen-Haushalt von niereninsuffizienten Patienten korrigiert werden kann. Vor dem Hintergrund, dass sich der Säure-Basen-Haushalt nicht durch Diffusion während der Dialyse korrigieren lässt, ist häufig eine Zufuhr von Puffersubstanzen erforderlich. Zum Ausgleichen des Gefälles zwischen Säuren und Basen eignet sich insbesondere Bikarbonat bzw. eine Bikarbonat-Pufferlösung. Die Bikarbonat-Pufferlösung wird für die Hämodialyse bevorzugt erst unmittelbar vor bzw. während der Behandlung hergestellt.

Die dafür bekannten kartuschenförmigen Behälter/ Kartuschen weisen grundsätzlich einen Fluideinlass-Anschlussport/ Konnektor und einen Fluidauslass-Anschlussport/ Konnektor auf, an welche jeweils eine Fluideinlassleitung und eine Fluidauslassleitung anbringbar sind. Ein kartuschenförmiger Behälter/ eine Kartusche, welcher/ welche ein Bikarbonatkonzentratpulver enthält, kann somit an eine Fluidquelle, beispielsweise eine Wasserquelle angeschlossen werden, wobei das den Behälter/ die Kartusche durchströmende Wasser das in dem Behälter/ der Kartusche befindliche Bikarbonatkonzentratpulver auflöst und dieses in die Dialysierflüssigkeit ausschwemmt.

Dabei ist es unter anderem aus der EP 0 532 835 B1 oder der DE 29 718 407 U1 bekannt, innerhalb des kartuschenförmigen Behälters/ der Kartusche ein Sieb/ einen Filter vorzusehen, welches/ welcher ungelöste Pulver-/ und/ oder Feststoffpartikel, wie etwa Bikarbonat, Natriumchlorid oder Ähnliches, zurückhalten und somit ein Austreten von Feststoff-/ bzw. Pulverpartikeln im ungelösten Zustand aus dem Behälter/ der Kartusche verhindern kann. In beiden Druckschriften wird die genaue bzw. exakte Ausgestaltung des Filters jedoch nicht beschrieben.

Aus der EP 1 610 842 B1 ist eine mögliche Ausbildung eines in einem kartuschenförmigen Behälter/ einer Kartusche angebrachten Filters/ Siebs bekannt. Der darin offenbarte Filter ist an einer Bikarbonatkartusche montierbar/ anbringbar und mit schlitzförmigen Öffnungen ausgestaltet. Eine Ausbildung von Filteröffnungen als Schlitze hat jedoch den Nachteil, dass dadurch keine ausreichend große Durchtrittsfläche bei gleichzeitiger Gewährleistung der Stabilität bereitgestellt wird.

Insbesondere können Schlitze nicht in beliebiger Anzahl angeordnet/ vorgesehen werden, ohne die Stützgeometrie und damit die Stabilität des Filters/ des Siebs beträchtlich zu schwächen. Herstellprozessbedingt wird bei einem Filter/ einem Sieb eine Stützgeometrie benötigt, welche insbesondere durch die Ausbildung der Öffnungen, deren Abstand voneinander und Anordnung zueinander sowie dem Anteil der Durchtrittsfläche bezogen auf die Gesamtfläche des Filters/ des Siebs vorgegeben ist.

Vor allem bei einem kleinen Filterdurchmesser ist nur eine äußerst begrenzte Anzahl an Schlitzen möglich, wenn gleichzeitig noch eine ausreichende Stützgeometrie bereitgestellt werden soll. Dabei hat sich insbesondere die naturgemäße geradlinige bzw. lineare Ausbildung von Schlitzen als nachteilhaft erwiesen. Außerdem hat sich gezeigt, dass Schlitze das Bikarbonat oft nicht in optimaler Weise in dem kartuschenförmigen Behälter/ der Kartusche zurückhalten können.

Ein kartuschenförmiger Behälter gemäß dem Oberbegriff des Anspruchs 1 ist in der EP 2 832 339 A1 offenbart. Siebartige Filtereinrichtungen sind in der US 2014/0124434 A1, der WO 2005/118485 A1 und der EP 0 019 794 A1 offenbart.

Vor diesem Hintergrund ist es Aufgabe der Erfindung, diese Nachteile aus dem Stand der Technik zu vermeiden oder wenigstens zu mildern. Insbesondere soll ein Filter/ ein Sieb bereitgestellt werden, welcher/ welches ungelöste Feststoffe bzw. Partikel effektiv zurückhält, gleichzeitig einen Durchfluss der Lösung durch den Filter/ das Sieb verbessert und zudem eine ausreichende mechanische Stabilität gewährleistet. Außerdem soll die Filtereinrichtung in einfacher Weise in einen kartuschenförmigen Behälter/ eine Kartusche integrierbar sein.

Diese Aufgabe wird durch einen kartuschenförmigen Behälter/ eine Kartusche nach Anspruch 1 gelöst. Vorteilhafte Weiterbildungen bzw. Ausführungsformen sind in den Unteransprüchen beansprucht und/oder werden nachfolgend erläutert.

Die Erfindung betrifft zunächst einen kartuschenförmigen Behälter/ eine Kartusche für eine extrakorporale Blutbehandlungsmaschine, welcher/ welche zumindest einen einen Fluideinlass und/oder einen Fluidauslass bildenden Anschlussport/ Konnektor und zumindest eine an dem zumindest einen Anschlussport angeordnete, siebartige Filtereinrichtung, insbesondere wie voranstehend beschrieben, aufweist, welche zum Zurückhalten von sich in dem Behälter/ der Kartusche befindlichen ungelösten Pulver-/ und/oder Feststoffpartikeln ausgelegt ist. Die siebartige Filtereinrichtung weist mindestens zwei Öffnungen auf, welche jeweils aus mindestens zwei zusammenhängenden Teilausnehmungen/ Öffnungsabschnitten zusammengesetzt sind, wobei ein nicht linearer/ nicht geradliniger Öffnungsverlauf in einem Übergangsbereich/ -abschnitt zwischen mindestens zwei der Teilausnehmungen vorliegt und jede einzelne Öffnung somit in ihrer Gesamtheit nicht linear/ nicht geradlinig verlaufend ausgebildet ist.

In anderen Worten wird erfindungsgemäß ein Filter/ ein Sieb für einen kartuschenförmigen Behälter/ eine Kartusche bereitgestellt, welcher/ welches ungelöstes Pulver sowie Feststoffe in dem Behälter/ der Kartusche zurückhalten soll. Der Filter/ das Sieb weist mindestens zwei Öffnungen auf, welche wiederum aus mindestens zwei partiellen Ausnehmungen/ Teilausnehmungen/ Öffnungsabschnitten zusammengesetzt sind.

Die Öffnungen sind aus einer Vielzahl von Teilausnehmungen zusammengesetzt, die sowohl identisch als auch nicht identisch ausgestaltet sein können. Dabei sind unter identischen Teilausnehmungen Abschnitte der Öffnungen zu verstehen, welche jeweils eine identische bzw. gleiche Form sowie identische bzw. gleiche äußere Abmessungen aufweisen. Nicht identische Teilausnehmungen hingegen unterscheiden sich in ihrer Form und/ oder ihren Abmessungen. Zur Bildung einer Öffnung können die Teilausnehmungen beispielsweise zueinander verdreht angeordnet sein, wobei sowohl nur identische als auch nur nicht identische als auch identische und nicht identische Teilausnehmungen jeweils kombiniert eine Öffnung ausbilden können. Eine Teilausnehmung geht grundsätzlich in einem Übergangsbereich/ -abschnitt in eine andere Teilausnehmung über.

Die Teilausnehmungen gehen somit in einem Übergangsbereich/ -abschnitt ineinander über und bilden zusammen eine durchgehende Gesamtöffnung bzw. Gesamtausnehmung. Zumindest zwei der die Öffnung ausbildenden Teilausnehmungen weisen in ihrem Übergangsbereich/ -abschnitt einen nicht linearen/ nicht geradlinigen Öffnungsverlauf/ Verlauf auf. Es hat sich herausgestellt, dass, wenn jede einzelne der Öffnungen in ihrer Gesamtheit nicht linear verlaufend bzw. sich nicht linear erstreckend/ ausbreitend ausgebildet ist, eine verfügbare Durchtrittsfläche, insbesondere bei einem kleinen verfügbaren Filterdurchmesser, insbesondere im Vergleich zu den im Stand der Technik verwendeten linearen bzw. geradlinigen Schlitzen, in großem Maße erhöht werden und somit der Durchfluss der Pufferlösung durch das Sieb verbessert werden kann.

Unter nicht linear verlaufenden Öffnungen sind somit erfindungsgemäß Öffnungen bzw. Ausnehmungen zu verstehen, welche zwischen zwei Endabschnitten zumindest abschnittsweise gekrümmt/ kurvig/ gewölbt bzw. abgeknickt ausgebildet sind und damit eine Änderung in ihrer Raumrichtung durchlaufen.

Bevorzugt können die Öffnungen in ihrer Gesamtheit näherungsweise symmetrisch, insbesondere achsensymmetrisch, ausgebildet sein. Es hat sich herausgestellt, dass gerade durch eine Merkmalskombination aus nicht linear verlaufenden sowie näherungsweise symmetrisch ausgebildeten Öffnungen ein Durchfluss der Lösung durch den Filter/ das Sieb verbessert und gleichzeitig eine verbesserte Stabilität der Stützstruktur der Filtereinrichtung bereitgestellt werden kann.

Die zumindest zwei zusammenhängenden Teilausnehmungen erstrecken sich von einem gemeinsamen Punkt in zumindest zwei unterschiedliche bzw. nicht parallele Richtungen weg. Dabei haben sich insbesondere V-förmige, Y-förmige, X- bzw. kreuzförmige oder andere stern- bzw. strahlenförmige Öffnungen als vorteilhaft erwiesen.

Gemäß einem vorteilhaften Ausführungsbeispiel sind die Öffnungen sternförmig und/oder strahlenförmig ausgebildet und weisen zumindest zwei, vorzugsweise drei, vier, fünf oder mehr, Strahlen und/oder Zacken auf, welche sich jeweils von einem gemeinsamen Punkt/ Übergangspunkt/ Schnittpunkt, insbesondere Mittelpunkt, radial nach außen erstrecken. Der gemeinsame Punkt/ Übergangspunkt/ Schnittpunkt kann aber auch außermittig angeordnet sein.

In vorteilhafter Weise spannen die Öffnungen dabei jeweils einen Kreis auf und/ oder ist eine Dimension/ Erstreckung der Öffnungen in einer ersten Richtung identisch mit einer Dimension/ Erstreckung der Öffnungen in einer zweiten, vorzugsweise zu der ersten Richtung senkrechten, Richtung.

In anderen Worten werden gerade durch die kompakte, nicht langgestreckte Form der Öffnungen, welche aus Teilausnehmungen, die sich von einem gemeinsamen Punkt in nicht parallele Richtungen weg erstrecken, zusammengesetzt sind und welche einen Kreis und/ oder ein Quadrat aufspannen, die Vorteile der vorliegenden Erfindung erreicht. Insbesondere dienen diese Merkmale einem verbesserten Fluiddurchfluss durch den Filter/ das Sieb und in Verbindung mit einer achsensymmetrischen Ausbildung der Öffnungen und/ oder einer gleichmäßigen Verteilung der Zacken/ Strahlen um einen gemeinsamen Mittelpunkt ebenso einer verbesserten Stabilität. Es wird erfindungsgemäß somit eine stabilere Stützstruktur und gleichzeitig eine vergrößerte Durchtrittsfläche bereitgestellt.

Alternativ oder zusätzlich können die Öffnungen wellenförmig und/oder mäanderförmig und/oder gezackt und/oder hakenförmig und/oder punktförmig ausgebildet sein. Denkbar sind erfindungsgemäß ferner stufenförmige, punktförmige, rechteckförmige, halbkreisförmige oder parabelförmige Öffnungen. Außerdem können Öffnungen in Form von unterbrochenen Kreisen und/oder unterbrochenen Quadraten vorgesehen sein.

Es ist von Vorteil, wenn die Öffnungen an der, insbesondere kreisförmigen, Filtereinrichtung zumindest annähernd gleichmäßig verteilt angeordnet sind und/oder mit ihren Mittel- oder Übergangspunkten auf einem von mindestens zwei zueinander konzentrischen Kreisen liegen, wobei insbesondere eine Anzahl von sich auf einem der konzentrischen Kreise befindlichen Öffnungen von einer radialen Innenseite zu einer radialen Außenseite hin zunehmend ist und/ oder eine Größe der Öffnungen umso größer ist, je weniger konzentrische Kreise, auf welchen die Öffnungen angeordnet sind, vorgesehen sind.

Es ist erfindungsgemäß insbesondere wichtig, die Vielzahl an vorgesehenen Öffnungen in geeigneter Weise auf der kreisförmigen Filtereinrichtung zu verteilen. Nur durch eine gleichmäßige Verteilung der Öffnungen einhergehend mit einer geeigneten Form der Öffnungen kann nämlich eine ausreichende Stabilität auch bei vergrößerter Durchtrittsfläche erreicht werden. Es hat sich dabei herausgestellt, dass es von Vorteil ist, die Ausnehmungen mit ihren Mittel- oder Übergangspunkten auf einem von zwei, drei, vier oder mehr vorgesehenen konzentrischen Kreisen anzuordnen und vorzugsweise entlang des Kreisumfangs eines konzentrischen Kreises herum jeweils gleichmäßig, das heißt mit einem gleichmäßigen Winkelabstand, zu verteilen. Die Öffnungen sind vorzugsweise symmetrisch an der Filtereinrichtung angeordnet, können jedoch auch asymmetrisch angeordnet sein.

Gemäß einem bevorzugten Ausführungsbeispiel der vorliegenden Erfindung ist eine Vielzahl von stern- und/oder strahlenförmigen, identischen Öffnungen/ Ausnehmungen mit vier oder fünf Strahlen/ Zacken an der siebartigen Filtereinrichtung vorgesehen, welche mit ihren Mittelpunkten/ Übergangspunkten auf zwei zueinander konzentrischen Kreisen angeordnet sind. Bevorzugt sind die Öffnungen dabei gleichmäßig verteilt angeordnet.

Es ist zweckmäßig, wenn eine Anordnung und/ oder eine Anzahl und/oder eine Geometrie der Öffnungen in Abhängigkeit von dem Durchmesser des Filters/ des Siebs ausgewählt wird.

Die erfindungsgemäße Filtereinrichtung weist einen sehr geringen bevorzugten Durchmesser auf. Dadurch, dass erfindungsgemäß keine Schlitze, sondern die voranstehend beschriebenen Öffnungen verwendet werden, kann auch bei einem kleinen Durchmesser des Filters/ des Siebs eine geeignete Gesamtdurchtrittsfläche der Öffnungen unter Einhaltung der Stabilitätsanforderungen bereitgestellt werden. Die Anordnung, Anzahl bzw. Geometrie der Öffnungen ist somit erfindungsgemäß im Hinblick auf ein Verhältnis von Durchtrittsfläche zu Gesamtfläche bei gleichzeitiger Einhaltung der Stabilitätsanforderungen optimiert.

Natürlich ist ein Einhalten der Stabilitätsanforderungen auch immer von der vorliegenden Tiefe der Filtereinrichtung abhängig. Die Tiefe des Filters/ des Siebs ist erfindungsgemäß etwas größer oder gleich einer maximalen Breite der Teilausnehmungen der Öffnungen.

Es ist dabei erfindungsgemäß vorgesehen, dass die Teilausnehmungen der Öffnungen eine vorbestimmte maximale Breite, welche insbesondere durch die sich in dem Aufnahmebehälter befindlichen Pulver- und/oder Feststoffpartikel vorgegeben ist, nicht überschreiten. Die Teilausnehmungen der Öffnungen sind somit in anderen Worten bevorzugt schmal und/oder langgestreckt ausgebildet.

Ein vorteilhaftes Ausführungsbeispiel ist ferner dadurch gekennzeichnet, dass die Öffnungen umlaufend/ vollumfänglich abgerundete Außenkanten aufweisen. Abgerundete Außenkanten der Öffnungen haben sich insbesondere im Hinblick auf das Einhalten der Stabilitätsanforderungen als ebenfalls sehr vorteilhaft erwiesen. Abgerundete Außenkanten haben dabei den Vorteil, dass keine Kerbwirkung auftritt und die Öffnungen nicht einreißen.

Zusammengefasst stellt die vorliegende Erfindung einen Filter/ ein Sieb bereit, welcher/ welches eine geeignete Anzahl an Öffnungen mit einer spezifischen Geometrie und Anordnung zueinander aufweist. Insbesondere wird durch die Erfindung ein Filter/ ein Sieb zur Verfügung gestellt, welcher/ welches eine ausreichend große Durchtrittsfläche bei Gewährleistung der Stabilität bereitstellt und zudem ein Austreten von ungelösten Feststoffpartikeln aus dem Behälter/ der Kartusche verhindert. In anderen Worten wird erfindungsgemäß eine Durchtrittsfläche im Verhältnis zur Gesamtfläche optimiert. Der Filter/ das Sieb kann grundsätzlich einstückig/ einmaterialig/ einteilig/ integral/ stoffschlüssig mit dem Aufnahmebehälter ausgebildet sein oder formschlüssig in diesem, insbesondere in einem Anschlussport des Aufnahmebehälters, aufgenommen sein.

Bevorzugt ist die Filtereinrichtung einmaterialig und/oder einstückig und/oder integral und/oder einteilig mit dem Behälter/ der Kartusche hergestellt.

Insbesondere wird erfindungsgemäß der kartuschenförmige Behälter/ die Kartusche zusammen mit dem zumindest einen Anschlussport und der zumindest einen Filtereinrichtung in einem Prozessschritt im Spritzgussverfahren aus Kunststoff hergestellt. Der Behälter/ die Kartusche kann aus einem Grundkörperbauteil und einem Deckelbauteil bestehen, welche jeweils einen Anschlussport aufweisen. In diesem Fall wird sowohl das Grundkörperbauteil mit zugehörigem Anschlussport und Filtereinrichtung als auch das Deckelbauteil mit zugehörigem Anschlussport und Filtereinrichtung in jeweils einem Prozessschritt im Spritzgussverfahren hergestellt.

In anderen Worten wird die Filtereinrichtung in die Anschlussports des Behälters/ der Kartusche in einem einzigen Prozessschritt vollautomatisch integriert. Der Filter/ das Sieb muss somit erfindungsgemäß nicht in einem zweiten Prozessschritt in den/ die Anschlussport(s) des Behälters/ der Kartusche eingesetzt werden. Durch diese einteilige Ausbildung der Filtereinrichtung mit den Anschlussports wird eine Senkung von Material- und Herstellkosten erreicht. Außerdem wird durch die einteilige bzw. stoffschlüssige Integration der Filtereinrichtung in die Anschlussports/ Konnektoren eine mechanische Stabilität der Filtereinrichtung verbessert.

Die Erfindung wird nachfolgend mit Hilfe von Figuren weiter erläutert. Es zeigen:
- Fig. 1: eine erste bevorzugte Ausführungsform einer erfindungsgemäßen siebförmigen Filtereinrichtung;
- Fig. 2: eine zweite bevorzugte Ausführungsform der erfindungsgemäßen siebförmigen Filtereinrichtung;
- Fig. 3: eine Filtereinrichtung, welche weitere erfindungsgemäße Öffnungen aufweist;
- Fig. 4: eine Filtereinrichtung, welche ebenfalls weitere erfindungsgemäße Öffnungen aufweist;
- Fig. 5: einen kartuschenförmigen Behälter/ eine Kartusche, welcher/ welche zwei erfindungsgemäße Filtereinrichtungen aufweist;
- Fig. 6: eine achsensymmetrische erfindungsgemäße Öffnung bestehend aus fünf Teilausnehmungen;
- Fig. 7: eine punktsymmetrische erfindungsgemäße Öffnung bestehend aus vier Teilausnehmungen; und
- Fig. 8: eine asymmetrische erfindungsgemäße Öffnung bestehend aus vier nicht identischen bzw. unterschiedlichen Teilausnehmungen.

Die Figuren sind lediglich schematischer Natur und dienen ausschließlich dem Verständnis der Erfindung. Gleiche Elemente sind dabei mit denselben Bezugszeichen versehen. Die Merkmale der einzelnen Ausführungsbeispiele können untereinander ausgetauscht werden.

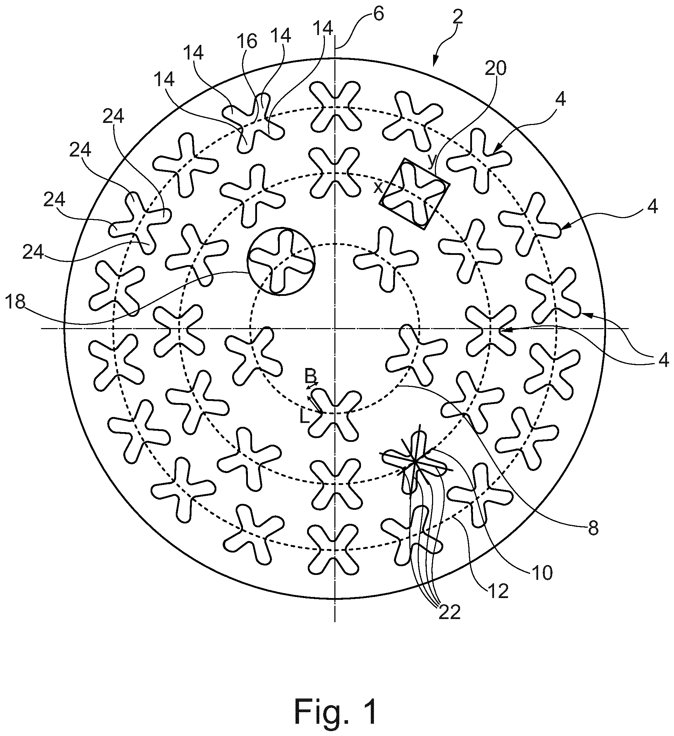

In Fig. 1 ist eine erste bevorzugte Ausführungsform einer siebförmigen Filtereinrichtung 2 gezeigt. Die Filtereinrichtung 2 weist 35 Öffnungen 4 auf, welche auf dem ersten Blick etwa gleichmäßig verteilt an der Filtereinrichtung 2 sowie achsensymmetrisch zu einer Filtersymmetrieachse 6 vorgesehen sind. Dabei liegen 5 der Öffnungen 4 auf einem Innenkreis 8, 12 der Öffnungen 4 auf einem Mittelkreis 10 und 18 der Öffnungen 4 auf einem Außenkreis 12. Das bedeutet auch, dass sich keine Öffnung 4 in einem Zentrum der Filtereinrichtung befindet. An den einzelnen Kreisen 8, 10, 12 sind die vorgesehenen Öffnungen 4 gleichmäßig, das heißt mit zueinander gleichem Winkelabstand, beabstandet. Der Kreise 8, 10, 12 sind zueinander konzentrisch.

Die Öffnungen 4 der Fig. 1 sind alle identisch ausgebildet und weisen jeweils eine Strahlenform/ Sternform auf. Die Öffnungen 4 bestehen jeweils aus vier zusammenhängenden, vorzugsweise identischen, Teilausnehmungen 14, welche zu einem gemeinsamen Mittelpunkt 16 zusammenlaufen bzw. sich von diesem in unterschiedliche Richtungen weg erstrecken. In einem Bereich um den gemeinsamen Mittelpunkt 16 liegen Übergangsbereiche/ -abschnitte 17 zwischen den Teilausnehmungen 14 vor. Zumindest die aneinander direkt angrenzenden, das heißt nicht gegenüberliegende, Teilausnehmungen 14 verlaufen in ihrem Übergangsbereich/ - abschnitt 17 nicht linear bzw. nicht geradlinig. Wie in Fig. 1 ersichtlich wird, spannen die Öffnungen einen Öffnungskreis 18 bzw. ein Öffnungsquadrat 20 auf. An dem Öffnungsquadrat 20 wird deutlich, dass eine Erstreckung x der Öffnungen 4 identisch bzw. gleich lang ist wie eine dazu senkrechte Erstreckung y der Öffnungen 4. Die Öffnungen 4 der Fig. 1 weisen jeweils vier Öffnungssymmetrieachsen 22 auf. Durch die Teilausnehmungen 14 der Fig. 1 werden pro Öffnung 4 vier Strahlen/ Zacken 24 definiert. Jede Teilausnehmung 14 weist eine Breite B und eine Länge L auf. Die Länge L ist dabei länger als die Breite B. Die Breite B darf eine vorbestimmte maximale Breite Bₘₐₓ nicht überschreiten, damit Pulver- bzw. Feststoffpartikel nicht durch die Öffnungen 4 gelangen können. Die Öffnungen 4 sind an ihren Außenkanten jeweils abgerundet gestaltet.

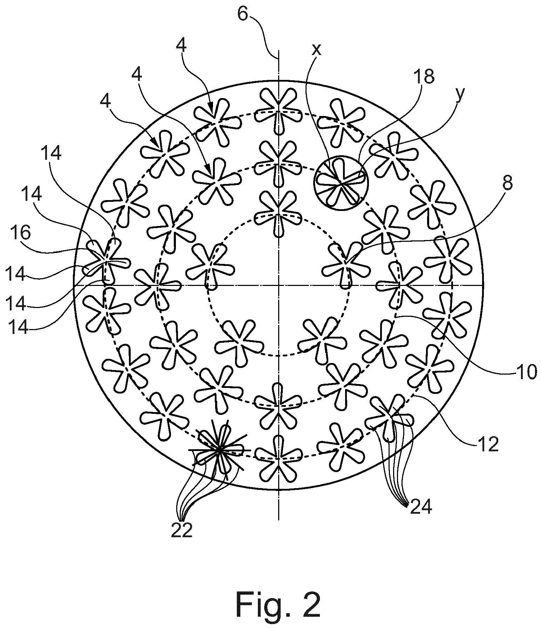

In Fig. 2 ist eine zweite bevorzugte Ausführungsform der siebförmigen Filtereinrichtung 2 dargestellt. Die Filtereinrichtung 2 weist wiederum 35 Öffnungen 4 auf. Die Anordnung der Öffnungen 4 zueinander ist identisch zu der in Fig. 1 vorgestellten Anordnung, weshalb eine Beschreibung nicht wiederholt wird. Nachfolgend wird lediglich auf die Unterschiede zwischen Fig. 1 und Fig. 2 eingegangen.

Die Öffnungen 4 der Fig. 2 sind ebenfalls alle identisch ausgebildet und weisen auch jeweils eine Strahlenform/ Sternform auf. Die Öffnungen 4 der Fig. 2 bestehen jedoch aus jeweils fünf zusammenhängenden, identischen Teilausnehmungen 14, welche zu einem gemeinsamen Mittelpunkt 16 zusammenlaufen bzw. sich von diesem in unterschiedliche Richtungen weg erstrecken. Wiederum liegen in einem Bereich um den gemeinsamen Mittelpunkt 16 Übergangsbereiche/ -abschnitte 17 zwischen den Teilausnehmungen 14 vor. Hier liegt in dem Übergangsbereich/ -abschnitt 17 zwischen zwei beliebigen der fünf Teilausnehmungen 14 ein nicht linearer bzw. nicht geradliniger Öffnungsverlauf vor. Wie in Fig. 2 ersichtlich wird, spannen die Öffnungen 4 der Fig. 2 einen Öffnungskreis 18 auf. Damit gilt auch für die Öffnungen 4 der Fig. 2, dass eine Erstreckung x der Öffnungen 4 gleich lang einer dazu senkrechten Erstreckung y der Öffnungen 4 ist. Die Öffnungen 4 der Fig. 2 weisen jeweils fünf Öffnungssymmetrieachsen 22 auf. Durch die Teilausnehmungen 14 der Fig. 2 werden pro Öffnung 4 fünf Strahlen/ Zacken 24 definiert. Ansonsten ist die Beschreibung zu Fig. 1 entsprechend auf Fig. 2 anzuwenden.

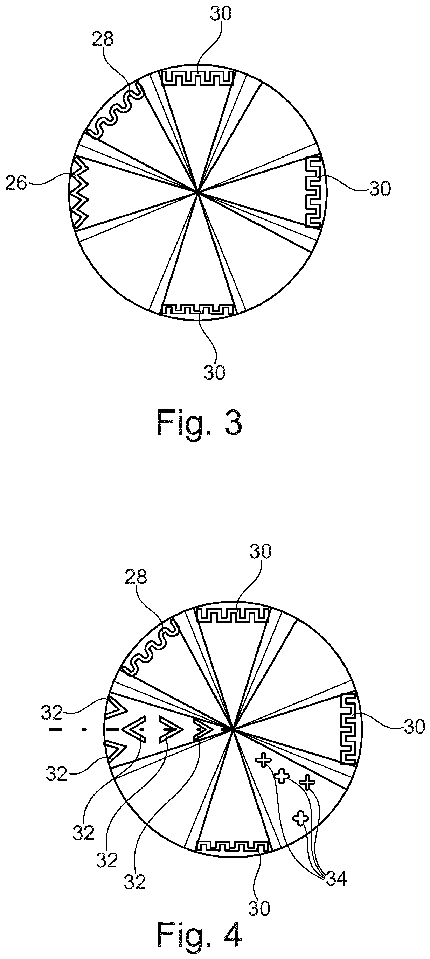

Die Fig. 3 und 4 zeigen lediglich verschiedene weitere Öffnungen, welche gemäß der vorliegenden Erfindung denkbar sind. Dabei sind in Fig. 3 eine gezackte Öffnung 26, eine wellenförmige Öffnung 28, sowie drei mäanderförmige Öffnungen 30 dargestellt. In Fig. 4 sind eine wellenförmige Öffnung 28, drei mäanderförmige Öffnungen 30, fünf hakenförmige Öffnungen 32, und vier kreuzpunktförmige Öffnungen 34 dargestellt. Die kreuzpunktförmigen Öffnungen 34 können abgerundete Kanten aufweisen oder auch nicht. Sämtliche in Fig. 3 und Fig. 4 dargestellten Öffnungen können an der erfindungsgemäßen Filtereinrichtung 2 gleichmäßig verteilt anstatt der sternförmigen/ strahlenförmigen Öffnungen der Fig. 1 und Fig. 2 vorgesehen sein. Auch können an einer Filtereinrichtung 2 verschiedenartige erfindungsgemäße Öffnungen vorgesehen sein. In anderen Worten müssen die Öffnungen nicht, wie dies in den Fig. 1 und 2 der Fall ist, alle identisch ausgebildet sein.

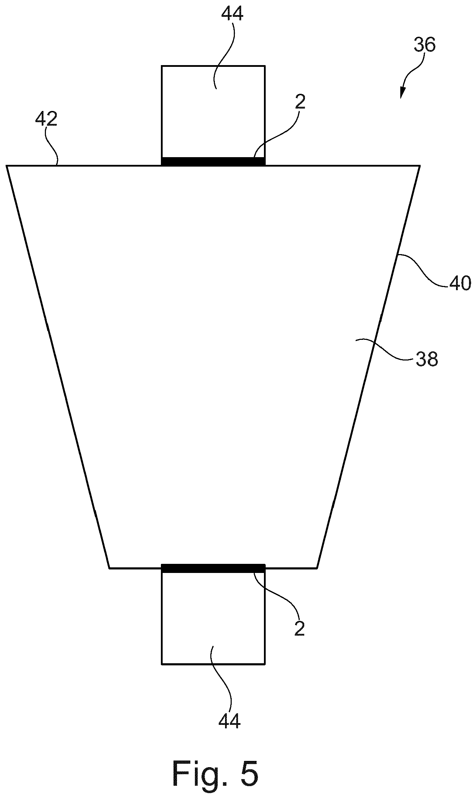

In Fig. 5 ist eine Kartusche 36 gezeigt. Diese setzt sich vorzugsweise aus einem Grundkörperbauteil 40 und einem Deckelbauteil 42 zusammen. Die Kartusche 36 weist an zwei gegenüberliegenden axialen Enden rohrstutzenförmige Anschlussports/ Konnektoren 44 auf, welche einen Fluideinlass und einen Fluidauslass bilden. An den beiden Anschlussports 44 sind jeweils Filtereinrichtungen 2 vorgesehen. Die Filtereinrichtungen 2 weisen dabei die in den Fig. 1 bis 4 gezeigten Öffnungen 4 auf und ermöglichen einen Fluiddurchfluss und ein Zurückhalten von ungelösten Partikeln. Das Deckelbauteil 42, der dazugehörige, in Fig. 5 obere, Anschlussport 44, sowie die dazugehörige, in Fig. 5 obere, Filtereinrichtung 2 sind als ein einstückiges Bauteil in einem Prozessschritt im Spritzgussverfahren hergestellt. Das Grundkörperbauteil 40, der dazugehörige, in Fig. 5 untere, Anschlussport, sowie die dazugehörige, in Fig. 5 untere, Filtereinrichtung 2 sind ebenso als ein einstückiges Bauteil in einem Prozessschritt im Spritzgussverfahren hergestellt.

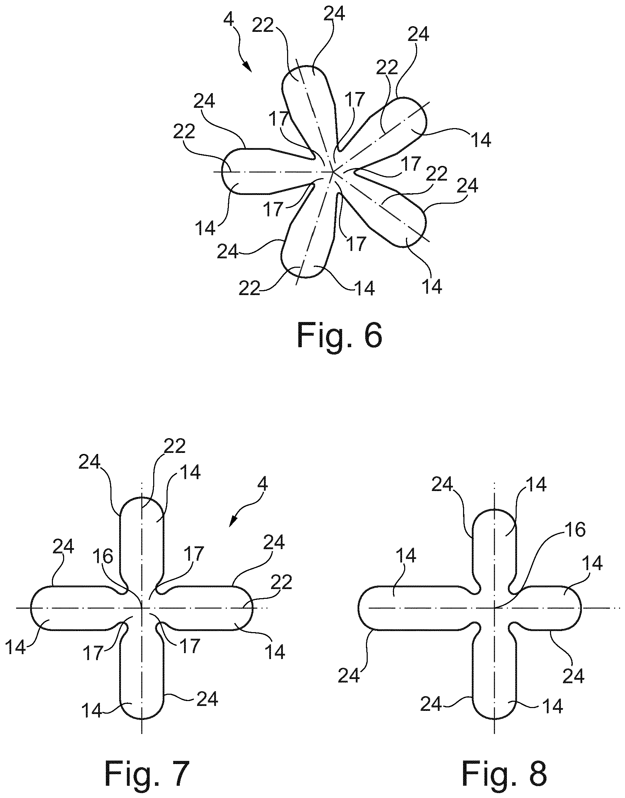

Fig. 6 zeigt eine erfindungsgemäße strahlen- bzw. sternförmige Öffnung 4, welche aus fünf Teilausnehmungen 14 bzw. fünf Strahlen/ Zacken 24 besteht. Die Öffnung 4 der Fig. 6 ist achsensymmetrisch ausgebildet und weist fünf Öffnungssymmetrieachsen 22 auf. Zwischen zwei beliebigen der fünf Teilausnehmungen 14 liegt in einem Übergangsbereich 17 ein nicht linearer Öffnungsverlauf vor. Eine Außenkontur der Öffnung 4 ist abgerundet ausgebildet.

Fig. 7 zeigt eine weitere erfindungsgemäße strahlen- bzw. sternförmige Öffnung 4, welche aus vier Teilausnehmungen 14 bzw. vier Strahlen/ Zacken 24 besteht. Die Öffnung 4 der Fig. 7 ist achsensymmetrisch mit zwei Symmetrieachsen 22 sowie punktsymmetrisch um den Mittelpunkt 16 ausgebildet. Zwischen zwei senkrecht aufeinander stehenden, benachbarten Teilausnehmungen 14 liegt in einem Übergangsbereich 17 jeweils ein nicht linearer Öffnungsverlauf vor. Eine Außenkontur der Öffnung 4 ist ebenso abgerundet. Ferner liegen an der Außenkontur zurückgenommene, abgerundete Übergänge zwischen zwei Teilausnehmungen 14 vor.

Fig. 8 zeigt eine weitere erfindungsgemäße strahlen- bzw. sternförmige Öffnung 4, welche aus vier Teilausnehmungen 14 bzw. vier Strahlen/ Zacken 24 besteht. Die Öffnung 4 der Fig. 8 ist asymmetrisch ausgebildet. Die vier Teilausnehmungen 14 weisen alle eine unterschiedliche Größe bzw. unterschiedliche äußere Abmessungen auf. Die Teilausnehmungen 14 sind somit nicht identisch, erstrecken sich jedoch, wie dies auch in den Ausführungsformen der Fig. 6 und der Fig. 7 der Fall ist, von einem gemeinsamen Schnittpunkt/ Übergangspunkt 16, welcher in diesem Fall kein Mittelpunkt ist, in zwei nicht parallele Richtungen weg. Eine Außenkontur der Öffnung 4 ist ebenso abgerundet. Ferner liegen an der Außenkontur auch zurückgenommene, abgerundete Übergänge zwischen zwei Teilausnehmungen 14 vor.

An der Filtereinrichtung 2 der vorliegenden Erfindung kann eine Vielzahl von identischen Öffnungen 4, jedoch auch eine Vielzahl von unterschiedlichen Öffnungen 4 vorliegen.

### Bezugszeichenliste

- 2: Filtereinrichtung
- 4: Öffnung
- 6: Filtersymmetrieachse
- 8: Innenkreis
- 10: Mittelkreis
- 12: Außenkreis
- 14: Teilausnehmung
- 16: Mittel-/ Übergangs-/ Schnittpunkt
- 17: Übergangsbereich/ -abschnitt
- 18: Öffnungskreis
- 20: Öffnungsquadrat
- 22: Öffnungssymmetrieachse
- 24: Strahlen/ Zacken
- 26: gezackte Öffnung
- 28: wellenförmige Öffnung
- 30: mäanderförmige Öffnung
- 32: hakenförmige Öffnung
- 34: kreuzpunktförmige Öffnung
- 36: kartuschenförmiger Behälter/ Kartusche
- 40: Grundkörperbauteil
- 42: Deckelbauteil
- 44: Anschlussport

## Patentansprüche

1. Kartuschenförmiger Behälter (36) für eine extrakorporale Blutbehandlungsmaschine mit mindestens einen einen Fluideinlass und/oder einen Fluidauslass bildenden Anschlussport (44) und mit mindestens einer an dem mindestens einen Anschlussport (44) angeordneten, siebartigen Filtereinrichtung (2), welche zum Zurückhalten von sich in dem Behälter (36) befindlichen ungelösten Pulver-/ und/ oder Feststoffpartikeln ausgelegt ist, wobei die siebartige Filtereinrichtung (2) mindestens zwei Öffnungen (4) aufweist, welche jeweils aus mindestens zwei zusammenhängenden Teilausnehmungen (14) zusammengesetzt sind, wobei ein nicht linearer Öffnungsverlauf in einem Übergangsbereich (17) zwischen mindestens zwei der Teilausnehmungen (14) vorliegt und jede einzelne Öffnung (4) somit in ihrer Gesamtheit nicht linear verlaufend ausgebildet ist, **dadurch gekennzeichnet, dass** sich die mindestens zwei zusammenhängenden Teilausnehmungen (14) von einem gemeinsamen Punkt (16) in zumindest zwei nicht parallele Richtungen weg erstrecken.

2. Kartuschenförmiger Behälter (36) nach Anspruch 1, **dadurch gekennzeichnet, dass** die Öffnungen (4) jeweils einen Kreis (18) aufspannen oder eine Erstreckung (x) der Öffnungen (4) in einer ersten Richtung identisch ist mit einer Erstreckung (y) der Öffnungen (4) in einer zweiten Richtung.

3. Kartuschenförmiger Behälter (36) nach Anspruch 1 oder 2, **dadurch gekennzeichnet, dass** die Öffnungen (4) sternförmig oder strahlenförmig ausgebildet sind und zumindest zwei Strahlen oder Zacken (24) aufweisen, welche sich jeweils von einem gemeinsamen Punkt (16) radial nach außen erstrecken.

4. Kartuschenförmiger Behälter (36) nach Anspruch 3, **dadurch gekennzeichnet, dass** der gemeinsame Punkt (16) ein Öffnungsmittelpunkt oder ein Übergangspunkt oder Schnittpunkt zwischen mindestens zwei zusammenhängenden Teilausnehmungen (14) ist.

5. Kartuschenförmiger Behälter (36) nach Anspruch 1 oder 2, **dadurch gekennzeichnet, dass** die Öffnungen (4) wellenförmig oder mäanderförmig oder gezackt oder hakenförmig oder punktförmig ausgebildet sind.

6. Kartuschenförmiger Behälter (36) nach einem der vorhergehenden Ansprüche, **dadurch gekennzeichnet, dass** die Öffnungen (4) an der Filtereinrichtung (2) zumindest annähernd gleichmäßig verteilt angeordnet sind oder mit ihren Mittelpunkten (16) auf einem von mindestens zwei zueinander konzentrischen Kreisen (8, 10, 12) liegen, wobei eine Anzahl von sich auf einem der konzentrischen Kreise (8, 10, 12) befindlichen Öffnungen (4) von einer radialen Innenseite zu einer radialen Außenseite hin zunehmend ist oder eine Größe der Öffnungen (4) umso größer ist, je weniger konzentrische Kreise (8, 10 ,12), auf welchen die Öffnungen (4) angeordnet sind, vorgesehen sind.

7. Kartuschenförmiger Behälter (36) nach einem der vorhergehenden Ansprüche, **dadurch gekennzeichnet, dass** die Teilausnehmungen (14) der Öffnungen (4) eine vorbestimmte maximale Breite (Bₘₐₓ), welche durch die sich in dem Behälter (36) befindlichen Pulver- und/oder Feststoffpartikel vorgegeben ist, nicht überschreiten.

8. Kartuschenförmiger Behälter (36) nach einem der vorhergehenden Ansprüche, **dadurch gekennzeichnet, dass** die Öffnungen (4) umlaufend abgerundete Außenkanten aufweisen.

9. Kartuschenförmiger Behälter (36) nach einem der vorhergehenden Ansprüche, **dadurch gekennzeichnet, dass** die Filtereinrichtung (2) einmaterialig oder einstückig oder integral oder einteilig mit dem Behälter (36) hergestellt ist.

## Claims

1. A cartridge-shaped container (36) for an extracorporeal blood treatment machine comprising at least one connecting port (44) forming a fluid inlet and/or a fluid outlet and at least one screen-style filter device (2) arranged on the at least one connecting port (44) and designed for withholding undissolved powder and/or solid particles present in the container (36), the screen-style filter device (2) comprising at least two openings (4), each of which being composed of at least two connected part recesses (14), wherein a non-linear run of the opening is provided in a transition area (17) between at least two of the part recesses (14) and thus each individual opening (4) in its entirety is designed to be extending in a non-linear manner, **characterized in that** the at least two connected part recesses (14) extend away from a common point (16) into at least two non-parallel directions.

2. A cartridge-shaped container (36) according to claim 1, **characterized in that** each of the openings (4) each span a circle (18) or an extension (x) of the openings (4) in a first direction identical to an extension (y) of the openings (4) in a second direction.

3. A cartridge-shaped container (36) according to claim 1 or 2, **characterized in that** the openings (4) are star-shaped or radiant and have at least two beams or spikes (24) each extending radially outward from a common point (16).

4. A cartridge-shaped container (36) according to claim 3, **characterized in that** the common point (16) is an opening center point or a transition point or an intersection point between at least two connected part recesses (14).

5. A cartridge-shaped container (36) according to claim 1 or 2, **characterized in that** the openings (4) are wave-shaped or meander-shaped or serrated or hook-shaped or dot-shaped.

6. A cartridge-shaped container (36) according to any of the preceding claims, **characterized in that** the openings (4) on the filter device (2) are arranged to be spread at least approximately evenly or the centers (16) thereof are located on one of at least two circles (8, 10, 12) concentric to each other, wherein a number of openings (4) located on one of the concentric circles (8, 10, 12) increases from a radial inside toward a radial outside or a size of the openings (4) is the larger, the fewer the concentric circles (8, 10, 12) on which the openings (4) are arranged are provided.

7. A cartridge-shaped container (36) according to any of the preceding claims, **characterized in that** the part recesses (14) of the openings (4) do not exceed a predetermined maximum width (Bₘₐₓ) which is predefined by the powder and/or solid particles present in the container (36).

8. A cartridge-shaped container (36) according to any of the preceding claims, **characterized in that** the openings (4) have rounded outer edges over their entire circumference.

9. A cartridge-shaped container (36) according to any of the preceding claims, **characterized in that** the filter device (2) is manufactured from one material or in one piece or integrally or in one part with the container (36).

## Revendications

1. Contenant en forme de cartouche (36) pour une machine de traitement extracorporel du sang comportant au moins un orifice de raccordement (44) formant une entrée de fluide et/ou une sortie de fluide et comportant au moins un dispositif de filtration de type tamis (2) disposé au niveau de l'au moins un orifice de raccordement (44), qui est conçu pour retenir des particules de poudre et/ou des particules solides non dissoutes se trouvant dans le contenant (36), dans lequel le dispositif de filtration de type tamis (2) présente au moins deux ouvertures (4) qui sont composées respectivement d'au moins deux évidements partiels en relation (14), dans lequel une évolution d'ouverture non linéaire se trouve dans une zone de transition (17) entre au moins deux des évidements partiels (14) et chaque ouverture individuelle (4) est ainsi formée en évoluant de façon non linéaire dans sa totalité, **caractérisé en ce que** les au moins deux évidements partiels en relation (14) s'étendent d'un point commun (16) dans au moins deux directions non parallèles.

2. Contenant en forme de cartouche (36) selon la revendication 1, **caractérisé en ce que** les ouvertures (4) couvrent respectivement un cercle (18) ou une étendue (x) des ouvertures (4) est identique dans une première direction à une étendue (y) des ouvertures (4) dans une seconde direction.

3. Contenant en forme de cartouche (36) selon la revendication 1 ou 2, **caractérisé en ce que** les ouvertures (4) sont en forme d'étoile ou en forme de rayons et présentent au moins deux rayons ou dentelures (24) qui s'étendent respectivement radialement vers l'extérieur depuis un point commun (16).

4. Contenant en forme de cartouche (36) selon la revendication 3, **caractérisé en ce que** le point commun (16) est un point central d'ouverture ou un point de transition ou un point d'intersection entre au moins deux évidements partiels en relation (14).

5. Contenant en forme de cartouche (36) selon la revendication 1 ou 2, **caractérisé en ce que** les ouvertures (4) sont de forme ondulée ou en méandres ou dentées ou en forme de crochets ou en forme de point.

6. Contenant en forme de cartouche (36) selon l'une quelconque des revendications précédentes, **caractérisé en ce que** les ouvertures (4) au niveau du dispositif de filtration (2) sont disposées réparties au moins approximativement de façon uniforme ou leurs points centraux (16) se trouvent sur l'un parmi au moins deux cercles concentriques l'un par rapport à l'autre (8, 10, 12), dans lequel un nombre d'ouvertures (4) se trouvant sur l'un des cercles concentriques (8, 10, 12) augmente depuis un côté intérieur radial vers un côté extérieur radial ou une grandeur des ouvertures (4) est d'autant plus grande que le nombre de cercles concentriques (8, 10, 12) prévu, sur lesquels sont disposées les ouvertures (4), est réduit.

7. Contenant en forme de cartouche (36) selon l'une quelconque des revendications précédentes, **caractérisé en ce que** les évidements partiels (14) des ouvertures (4) ne dépassent pas une largeur maximale prédéterminée (Bₘₐₓ) qui est prédéterminée par les particules de poudre et/ou solides qui se trouvent dans le contenant (36).

8. Contenant en forme de cartouche (36) selon l'une quelconque des revendications précédentes, **caractérisé en ce que** les ouvertures (4) présentent des bords extérieurs arrondis au niveau périphérique.

9. Contenant en forme de cartouche (36) selon l'une quelconque des revendications précédentes, **caractérisé en ce que** le dispositif de filtration (2) est fabriqué en un seul matériau ou en une seule pièce ou d'un seul tenant ou monobloc avec le contenant (36).
